# EUROPEAN PATENT SPECIFICATION

(11) **EP 2 203 740 B1**
(45) Date of publication and mention of the grant of the patent: **03.08.2011**
(21) Application number: 08842040.1
(22) Date of filing: 22.10.2008
(51) Int. Cl.: G01N 33/22, G01N 1/22, F23G 5/50, F23N 5/00

(54) **METHOD FOR DETERMINING THE FOSSIL FUEL CONTENT IN A FUEL STREAM, AS WELL AS AN INCINERATION FURNACE**
VERFAHREN ZUR BESTIMMUNG DES GEHALTS AN FOSSILEN BRENNSTOFFEN IN EINEM BRENNSTOFFSTROM SOWIE VERBRENNUNGSOFEN
PROCÉDÉ POUR DÉTERMINER LA TENEUR EN COMBUSTIBLE FOSSILE DANS UN COURANT DE COMBUSTIBLE, AINSI QU'UN FOUR D'INCINÉRATION

(30) Priority: 24.10.2007 NL 2000960
(43) Date of publication of application: 07.07.2010
(73) Proprietor: Stichting Energieonderzoek Centrum Nederland, 1755 LE Petten (NL)
(72) Inventor: BAKKER, Fredericus Petrus, NL-1948 AM Beverwijk (NL); GEUSEBROEK, Marco, NL-1755 RD Petten (NL)
(74) Representative: Ketelaars, Maarten F.J.M.
(86) International application number: PCT/NL2008/050662
(87) International publication number: WO 2009/054718

(56) References cited:
- EP-A- 1 829 951
- WO-A-02/06730

## Description

The present invention relates to a method for determining the fossil fuel content in a fuel stream fed to an incineration furnace according to the preamble of Claim 1.

Various kinds of material are fed to incineration furnaces, for the most part fossil fuels, that is to say fuels which are tens of thousands of years old and older. In particular in the case of waste incinerators, but also with other incineration furnaces, a fuel stream is also supplied which comprises fuels which are less old. Examples thereof are wood, rubber material and the like. These fuels are also referred to as biogen fuels.

For environmental reasons, it is desirable to limit the emission of the greenhouse gas CO₂ as much as possible, in particular the CO₂ which is generated during the combustion of fossil fuels. Combustion of carbon which has been converted to, for example, wood relatively recently is not considered to be polluting. After all, wood is only a few tens to hundreds of years old and is the result of the conversion of carbon dioxide from the atmosphere into carbon.

In certain countries, charges have even been introduced for the emission of fossil fuel, that is to say fuel which originated more than 10,000 years ago.

If a mixed fuel stream is fed to an incineration furnace, it is important to know which fraction of the supplied fuel is fossil and which fraction of the fuel is more recent.

One possibility of determining this is through the analysis of the stream which is supplied.

Such a method is inaccurate when only random checks are carried out. In addition, it requires highly experienced investigators and/or is complicated.

A method for producing fuel from waste is known. It uses sampling and the fossil fuel content is determined by combusting CO₂ and using the C-14 method.

WO-A-02/06730 describes a method for determining the relationship between fossil and non-fossil energy carriers in a fuel mixture.

EP 1829951 discloses a method for the determination of the fosil fuel component in a fuel stream.

It is an object of the present invention to provide a simplified method in which the ratio between fossil and biogen fuels can be determined accurately.

This object is achieved with the above-described method using the features of Claim 1.

According to the present invention, the amount of ¹⁴C which is present is used to analyze the composition of the waste gas. The CO₂ which is present therein partly consists of CO₂ originating from fossil fuels and partly of CO₂ originating from biogen fuels.

¹⁴C is a radioactive element having a half-life of approximately 5700 years, which means that within said period, half the ¹⁴C content of any organic material which does not take part in the carbon cycle will disappear. In other words, all ¹⁴C will have disappeared within 60,000 years if the respective carbon is/was stored underground. In other words, in fossil fuels the ¹⁴C content is zero while in more recent biogen fuels it is approximately 1 ppt (10⁻¹²).

By determining the ¹⁴C content, the amount of non-fossil and biogen fuel, respectively, is known. If the total CO₂ percentage is known, the amount of fossil fuel in a sample can be calculated in a simple manner. Using the results, it is possible to determine, for example, charges and the like.

The accuracy of the method can be improved if a relationship is established between the sampled amount and the amount of discharged gas. This applies in particular in the case of varying amounts of discharged gas. This relationship is preferably linear, that is to say that the variation in the sampling is directly proportional to the variation in the amount of discharged gas.

There are various techniques for determining ¹⁴C. The most important technique which can be used in practice with the above-described method is the liquid scintillation counting (LSC) which allows very accurate measurements, for example having a relative standard deviation of less than 1% with samples which contain at least 25% of biogen mass. However, it is also possible to obtain good results with very low contents of non-fossil fuel, that is to say 5% or less. Other methods for determining ¹⁴C are β-ionization (gas burners) and accelerated mass spectrometry (AMS). For determining the total stream of waste gas or flue gas, a mass flow monitor can be used.

The invention also relates to a system according to claim 5.

Such an incineration furnace may, for example, comprise the furnace of a household waste incineration furnace. Other applications, such as in the production of cement and in power plants, are also possible.

The invention also relates to an assembly comprising an incineration furnace as described above in combination with a ¹⁴C-determining device. The ¹⁴C-determining device will generally be a laboratory which is situated at a distance from the incineration furnace. The sampling container can be removed at regular intervals from the flue gas outlet and replaced with another sampling container, with the first sampling container being taken to the ¹⁴C-determining device.

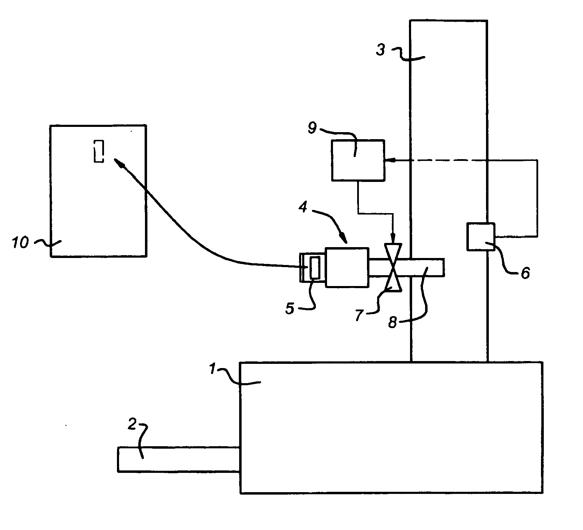

The invention will be described below with reference to an exemplary embodiment illustrated in the drawing.

Therein, the single figure diagrammatically shows an embodiment of the present invention.

In the figure, an incineration furnace is denoted by reference numeral 1. As has been indicated above, this may comprise any kind of incineration furnace, but is preferably an incineration furnace which can be fed with fuel streams of varying composition. Examples are household waste incineration furnaces and furnaces which are used in calcination, for example for preparing cement

The incineration furnace 1 comprises an inlet 2 for fuel. This fuel may comprise fossil fuel and non-fossil fuel. Examples of this non-fossil or biogen fuel are wood, packaging materials, paper, household vegetable waste, manure, slaughter waste, certain kinds of plastics, (car) tyres and the like.

The incineration furnace is provided with a chimney or outlet 3. The chimney contains a sampling device 4 and a mass flow meter 6. The sampling device comprises an inlet 8 in which a valve 7 and a sample container 5 are arranged.

At a distance from the waste incineration furnace, a sample-determining device is provided, which is denoted overall by reference numeral 10. The latter may, for example, be situated in a laboratory and is capable of determining ¹⁴C by means of the liquid scintillation method.

A valve control 9 is present which controls opening and closing of the valve 7 based on the signal originating from the mass flow meter 6.

The above-described device functions as follows:
When burning fuel in the incineration furnace 1, a sample is taken continually or in any other intermittent way and placed in sample container 5. Sample container 5 is periodically replaced and the sample obtained using sample container 5 is analysed for ¹⁴C in analyzing device 10. Depending on the amount of gas which flows through the chimney 3, and which is determined by the mass flow meter, the shut-off valve 17 is opened proportionally. That is to say, when a large amount of waste gas flows through, a large amount of material passes through the inlet 8. As a result of this proportional method, it is possible to obtain very accurate measurement results. This is particularly important when the amount of gas which flows through the chimney 3 varies.

Upon reading the above, those skilled in the art will immediately be able to think of variants which are within the scope of the present invention. Thus, it is possible to design the sample-determining device 10 in another manner. All that is important is that the latter is able to determine the biogen and fossil carbon content using the ¹⁴C method.

Depending on the application, either the fossil carbon content or the non-fossil carbon content, or both, are determined.

The above-described variants are obvious to those skilled in the art upon reading the above and are within the scope of the attached claims.

## Claims

1. Method for determining the fossil fuel content in a fuel stream fed to an incineration furnace (1), said method comprising sampling the waste gas stream, determining the carbon content in the CO₂ which originates from non-fossil fuel, using a ¹⁴C method for determining the proportion of non-fossil carbon, **characterized in that** said sampling comprises means (6) for determining the total amount of waste gases flowing through the incineration outlet (3) and, depending on said total amount of waste gases, measured by said determing means (6) activating-valve control means (9, 7) in response to the measured amount of total gases flowing through said outlet (3) allowing for accurate and proportional waste gas sampling by said sampling means (5).

2. Method according to Claim 1, wherein said fuel stream comprises portions of waste material.

3. Method according to one of the preceding claims, wherein the size of the sample is directly proportional to the size of the total waste gas stream.

4. Method according to one of the preceding claims, wherein a mass flow monitor is used for measuring the total waste gas stream.

5. C14- determination system for determing the amount of non-fossil and biogen fuel respectively in an incineration furnace (1), said incernation furnace comprising a ¹⁴Cinc-determining device (IC) and an inlet (2) for a fuel stream and an outlet (3) for the discharge of waste gases, wherein a sampling device (4) is arranged in said outlet, said sampling device is embodied to determine the non-fossil fuel content of the fuel stream and comprises a sampling container (5), having an inlet which can be connected to said outlet (3) for receiving said waste gases, **characterized in that** said sampling device is provided with a valve (7) for said inlet (8) and a valve control (9), wherein a sensor (6) is provided in said outlet which is connected to said valve control for determining the total amount of waste gases, wherein said valve control controls the size of the valve opening to said inlet depending on the total waste gas stream which has been measured by the sensor.

6. System according to Claim 5, wherein said incineration furnace comprises a household waste incineration furnace.

7. System according to Claim 5 or 6, wherein said incineration furnace comprises a part of a cement-producing device.

8. Assembly comprising a system furnace according to one of Claims 6-8, wherein said ¹⁴C-determining device is embodied to accommodate said sampling container (5).

9. Assembly according to Claim 8, wherein said ¹⁴C-determining device comprises a liquid scintillation device.

## Patentansprüche

1. Verfahren zur Bestimmung des fossilen Brennstoffgehaltes in einem zu einem Verbrennungsofen (1) geleiteten Brennstoffstrom, wobei das Verfahren eine Probenentnahme des Abgasstromes, eine Bestimmung des Kohlenstoffgehaltes in dem von dem nicht fossilen Brennstoff stammenden CO₂ unter Verwendung eines C¹⁴-Verfahrens umfasst, um den Anteil des nicht fossilen Kohlenstoffs zu bestimmen, **dadurch gekennzeichnet, dass** die Probenentnahme Mittel (6) zur Bestimmung der Gesamtmenge des durch den Verbrennungsauslass (3) strömenden Abgases und, in Abhängigkeit der durch die Bestimmungsmittel (6) gemessenen Gesamtmenge der Abgase, das Aktivieren von Ventilsteuermitteln (9, 7) als Folge der durch den Auslass (3) strömenden Menge an Gesamtgasen umfasst, um eine genaue und proportionale Abgasprobeentnahme durch die Probeentnahmemittel (6) zu ermöglichen.

2. Verfahren nach Anspruch 1, **dadurch gekennzeichnet, dass** der Brennstoffstrom Anteile von Abfallstoffen umfasst.

3. Verfahren nach einem der vorhergehenden Ansprüche, wobei die Größe der Probe direkt proportional zu der Größe des gesamten Abgasstromes ist.

4. Verfahren nach einem der vorhergehenden Ansprüche, wobei eine Massendurchflussüberwachung zur Messung des gesamten Abgasstromes verwendet wird.

5. C¹⁴-Bestimmungssystem zur Bestimmung der Menge nicht fossiler bzw. biogener Brennstoffe in einem Verbrennungsofen (1), wobei der Verbrennungsofen eine C¹⁴-Bestimmungseinrichtung (10) und einen Einlass (2) für einen Brennstoffstrom und einen Auslass (3) zum Abführen von Abgasen umfasst, wobei eine Probenentnahmeeinrichtung (4) in dem Auslass angeordnet ist, die Probenentnahmevorrichtung so ausgeführt ist, dass der nicht fossile Brennstoffgehalt des Brennstoffstroms bestimmbar ist und einen Probenentnahmebehälter (5) mit einem Einlass umfasst, der an den Auslass (3) zur Aufnahme der Abgase angeschlossen werden kann, **dadurch gekennzeichnet, dass** die Probenentnahmeeinrichtung mit einem Ventil (7) für den Einlass (8) und eine Ventilsteuerung (9) ausgerüstet ist, wobei ein Sensor (6) in dem Auslass angeordnet ist, der mit der Ventilsteuerung zur Bestimmung der Gesamtmenge von Abgasen verbunden ist, wobei die Ventilsteuerung die Größe der Ventilöffnung zu dem Einlass in Abhängigkeit des gesamten durch den Sensor gemessenen Abgasstromes steuert.

6. System nach Anspruch 5, wobei der Verbrennungsofen einen Verbrennungsofen für Haushaltsabfälle umfasst.

7. System nach Anspruch 5 oder 6, wobei der Verbrennungsofen einen Teil einer Zement herstellenden Vorrichtung umfasst.

8. Anordnung, umfassend ein Verbrennungssystem nach einem der Ansprüche 6 bis 8, wobei die C¹⁴-Bestimmungseinrichtung so ausgeführt ist, dass sie den Probenentnahmebehälter (5) aufnimmt.

9. Anordnung nach Anspruch 8, wobei die C₁₄-Bestimmungseinrichtung eine Flüssigkeitsszintillationsapparatur umfasst.

## Revendications

1. Procédé pour déterminer la teneur en combustible fossile dans un flux de combustible alimentant un four d'incinération (1), ledit procédé consistant à échantillonner le flux de gaz de combustion, déterminer la teneur en carbone dans le CO₂ qui provient du combustible non fossile en utilisant la méthode du carbone 14 pour déterminer la proportion de carbone non fossile, **caractérisé en ce que** ledit échantillonnage comprend des moyens (6) pour déterminer la quantité totale de gaz de combustion s'écoulant par l'orifice de sortie d'incinération (3), et en fonction de ladite quantité totale de gaz de combustion, mesurée par lesdits moyens de détermination (6), activer des moyens de commande de valve (9, 7) en réponse à la quantité mesurée des gaz totaux s'écoulant par ledit orifice de sortie (3) permettant l'échantillonnage précis et proportionnel des gaz de combustion par lesdits moyens d'échantillonnage (5).

2. Procédé selon la revendication 1, dans lequel ledit flux de combustible comprend des portions de déchets.

3. Procédé selon l'une des revendications précédentes, dans lequel la taille de l'échantillon est directement proportionnelle à la taille du flux total de gaz de combustion.

4. Procédé selon l'une des revendications précédentes, dans lequel un contrôleur de débit massique est utilisé pour mesurer le flux total de gaz de combustion.

5. Système de détermination du carbone 14 pour déterminer la quantité de combustible non fossile et biogène dans un four d'incinération (1), ledit four d'incinération comprenant un dispositif de détermination du carbone 14 (10) et un orifice d'entrée (2) pour un flux de combustible et un orifice de sortie (3) pour la décharge des gaz de combustion, dans lequel un dispositif d'échantillonnage (4) est agencé dans ledit orifice de sortie, ledit dispositif d'échantillonnage est mis en oeuvre pour déterminer la teneur en combustible non fossile du flux de combustible et comprend un récipient d'échantillonnage (5), ayant un orifice d'entrée qui peut être raccordé audit orifice de sortie (3) pour recevoir lesdits gaz de combustion, **caractérisé en ce que** ledit dispositif d'échantillonnage est doté d'une valve (7) pour ledit orifice d'entrée (8) et d'une commande de valve (9), dans lequel un capteur (6) est prévu dans ledit orifice de sortie qui est raccordé à ladite commande de valve pour déterminer la quantité totale des gaz de combustion, dans lequel ladite commande de valve commande la taille de l'ouverture de valve vers ledit orifice d'entrée en fonction du flux total de gaz de combustion qui a été mesuré par le capteur.

6. Système selon la revendication 5, dans lequel ledit four d'incinération comprend un four d'incinération de déchets domestiques.

7. Système selon la revendication 5 ou 6, dans lequel ledit four d'incinération comprend une partie d'un dispositif de production de ciment.

8. Ensemble comprenant un four de système selon l'une des revendications 6 à 8, dans lequel ledit dispositif de détermination de carbone 14 est mis en oeuvre de sorte à loger ledit récipient d'échantillonnage (5).

9. Ensemble selon la revendication 8, dans lequel ledit dispositif de détermination de carbone 14 comprend un dispositif de scintillation liquide.
